(19) Europäisches Patentamt
European Patent Office
Office européen des brevets

(11) EP 4 324 467 A1

(12) EUROPEAN PATENT APPLICATION
published in accordance with Art. 153(4) EPC

(43) Date of publication:
**21.02.2024 Bulletin 2024/08**

(21) Application number: **22787872.5**

(22) Date of filing: **28.02.2022**

(51) International Patent Classification (IPC):
***A61K 31/78*** (2006.01) ***A01N 61/00*** (2006.01)
***A01P 1/00*** (2006.01) ***A61P 31/14*** (2006.01)
***C08F 220/34*** (2006.01)

(52) Cooperative Patent Classification (CPC):
**A01N 61/00; A01P 1/00; A61K 31/78; A61P 31/14; C08F 220/34**

(86) International application number:
**PCT/JP2022/008180**

(87) International publication number:
**WO 2022/219944 (20.10.2022 Gazette 2022/42)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **16.04.2021 JP 2021069452**
**22.12.2021 JP 2021207663**

(71) Applicant: **Nippon Shokubai Co., Ltd.**
**Osaka-shi, Osaka 541-0043 (JP)**

(72) Inventors:
- **NAKANOSHO Masahiro**
  **Suita-shi, Osaka 564-0034 (JP)**
- **NISHINA Akira**
  **Suita-shi, Osaka 564-0034 (JP)**

(74) Representative: **Kraus & Lederer PartGmbB**
**Thomas-Wimmer-Ring 15**
**80539 München (DE)**

# (54) ANTI-CORONAVIRUS AGENT

(57) An object of the present invention is to provide an anticoronaviral agent and the like. Provided is an anticoronaviral agent comprising a polymer comprising at least one type of structural unit (a) selected from a structural unit represented by the following general formula (1):

[Chem. 1]

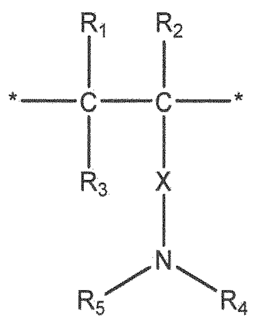

General formula (1)

a structural unit that is a salt of the general formula (1), and a structural unit represented by the following general formula (2):

EP 4 324 467 A1

[Chem. 2]

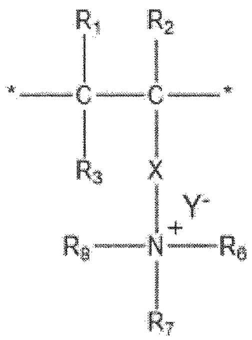

General formula (2)

wherein in the general formulae (1) and (2), $R_1$, $R_2$, and $R_3$ each independently represent a hydrogen atom or an alkyl group of 1 to 5 carbon atoms, $R_4$ to $R_8$ each independently represent a hydrogen atom or an organic group, X represents a divalent linking group, and $Y^-$ represents an anion.

**Description**

TECHNICAL FIELD

The present invention relates to an anticoronaviral agent and the like. More particularly, the present invention relates to an anticoronaviral agent comprising a specific polymer (polymer, copolymer, or amino group-containing copolymer).

BACKGROUND ART

Various antiviral agents have been proposed for the prevention of viral infections.
For example, Patent Literature 1 discloses a nanostructure comprising a block copolymer containing a specific ammonium moiety, which nanostructure can be used as an antiviral agent. Patent Literature 2 discloses a textile composition that comprises a combination of, for example, an amino acid silver, an amino acid zinc, a carboxyvinyl polymer, and a glutamic acid copper and has antiviral activity against influenza viruses.
Moreover, Non-Patent Literature 1 describes specific polyamide polyamine compounds having antiviral activity against various viruses.

CITATION LIST

Patent Literature

Patent Literature 1: JP-A 2021-011578
Patent Literature 2: JP-A 2020-012214

Non-Patent Literature

Non-Patent Literature 1:
Manuela D. et al. P6315-6319 Vol. 58 Oct. 2014 Antimicrobial Agents and Chemotherapy.

SUMMARY OF INVENTION

TECHNICAL PROBLEM

As described above, antiviral agents containing specific amino groups are known, but there has been a growing demand for antiviral agents that have excellent efficacy against coronaviruses, particularly the novel coronavirus and the like. An object of the present invention is to provide an anticoronaviral agent having an antiviral effect against the novel coronavirus and other viruses.

SOLUTION TO PROBLEM

The present inventors conducted extensive studies in order to achieve the above-mentioned object and finally arrived at the present invention.
That is, the present invention relates to the following.

[1] An anticoronaviral agent (anticoronaviral composition) comprising a polymer comprising at least one type of structural unit (a) selected from a structural unit represented by the following general formula (1):

[Chem. 1]

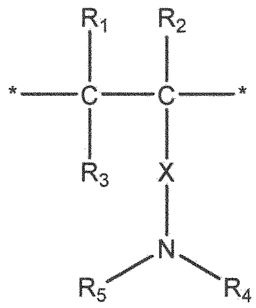

,

a structural unit that is a salt of the general formula (1), and a structural unit represented by the following general formula (2):

[Chem. 2]

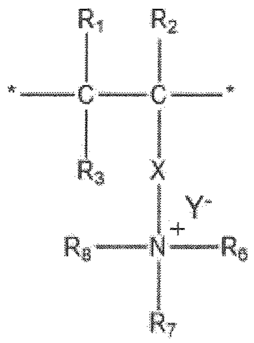

General formula (2)

, wherein in the general formulae (1) and (2), $R_1$, $R_2$, and $R_3$ each independently represent a hydrogen atom or an alkyl group of 1 to 5 carbon atoms, $R_4$ to $R_8$ each independently represent a hydrogen atom or an organic group, X represents a divalent linking group, and $Y^-$ represents an anion.

[2] The anticoronaviral agent according to the above [1], wherein the polymer further comprises a structural unit (b) having a Hildebrand solubility parameter of 35 $(MPa)^{1/2}$ or less.

[3] The anticoronaviral agent according to the above [1] or [2], wherein the polymer further comprises a structural unit (b) represented by the following general formula (3):

[Chem. 3]

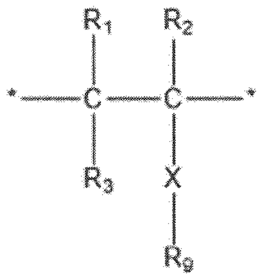

General formula (3)

,

and wherein in the general formula (3), $R_1$, $R_2$, and $R_3$ each independently represent a hydrogen atom or an alkyl group of 1 to 5 carbon atoms, X represents a divalent linking group, and $R_9$ represents a hydrocarbon group of 2 or more carbon atoms optionally having a substituent.

[4] The agent according to any one of the above [1] to [3], wherein the polymer has a Hildebrand solubility parameter of 8 to 30 $(MPa)^{1/2}$.

[5] The agent according to any one of the above [1] to [4], wherein the polymer further comprises a structural unit (b) having a Hildebrand solubility parameter of 32 $(MPa)^{1/2}$ or less, and wherein the polymer has a Hildebrand

solubility parameter of 10 to 25 $(MPa)^{1/2}$.

[6] The agent according to any one of the above [2] to [5], wherein the structural unit (a) comprises a structural unit derived from an N-mono- or di-alkylaminoalkyl (meth)acrylate, and wherein the structural unit (b) comprises a structural unit derived from a (meth)acrylic acid alkyl ester.

[7] The agent according to any one of the above [1] to [6], wherein the amount of the structural unit (a) in the polymer is 30% by mass or more and the amount of the structural unit (b) in the polymer is 1% by mass or more relative to the total amount of all the structural units, which is assumed as 100% by mass.

[8] The agent according to any one of the above [1] to [7], wherein the amount of the structural unit (a) in the polymer is 50% by mass or more and the amount of the structural unit (b) in the polymer is 5% by mass or more relative to the total amount of all the structural units, which is assumed as 100% by mass, and wherein the polymer has a Hildebrand solubility parameter of 12 to 23 $(MPa)^{1/2}$.

[9] The agent according to any one of the above [1] to [8], wherein the polymer is present at a concentration of 0.01% by mass or more.

[10] The agent according to any one of the above [1] to [9], wherein the agent is in the form of an aqueous solution [e.g., an aqueous solution having a pH of 2 to 11 (e.g., 4 to 9)] containing the polymer.

[11] A method for inactivating (killing) a coronavirus and/or inhibiting coronavirus growth using the agent (or composition) according to any one of the above [1] to [10].

Specifically, the present invention may be an anticoronaviral agent comprising a polymer (polymer, copolymer, or amino group-containing copolymer) having at least one type of structural unit (a) [structural unit (a) derived from an amino group-containing monomer] selected from a structural unit represented by the general formula (1) below, a structural unit that is a salt (e.g., a salt with an acid) of the general formula (1) below, and a structural unit represented by the general formula (2) below.

In particular, the present invention may be an anticoronaviral agent comprising a polymer (polymer, copolymer, or amino group-containing copolymer) having at least one type of structural unit (a) [structural unit (a) derived from an amino group-containing monomer] selected from a structural unit represented by the general formula (1) below, a structural unit that is a salt (e.g., a salt with an acid) of the general formula (1) below, and a structural unit represented by the general formula (2) below and an additional structural unit (b) represented by the general formula (3) below [structural unit (b) derived from a monomer containing a hydrocarbon group of 2 or more carbon atoms].

[Chem. 4]

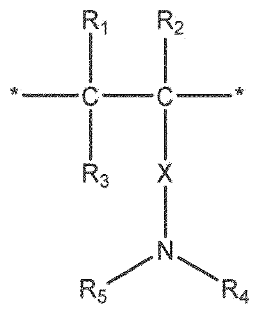

General formula (1)

[Chem. 5]

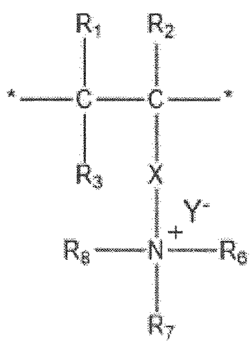

General formula (2)

In the general formulae (1) and (2), $R_1$, $R_2$, and $R_3$ each independently represent a hydrogen atom or an alkyl

group of 1 to 5 carbon atoms, $R_4$ to $R_8$ each independently represent a hydrogen atom or an organic group of 1 to 12 carbon atoms, X represents a divalent linking group, and $Y^-$ represents an anion. The asterisk represents an atom contained in another structural unit of the same or different type to which the structural unit represented by the general formula (1) or (2) is bound.

[Chem. 6]

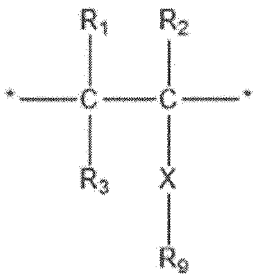

General formula (3)

In the general formula (3), $R_1$, $R_2$, and $R_3$ each independently represent a hydrogen atom or an alkyl group of 1 to 5 carbon atoms, X represents a divalent linking group, and $R_9$ represents a hydrocarbon group of 2 or more carbon atoms optionally having a substituent. The asterisk represents an atom contained in another structural unit of the same or different type to which the structural unit represented by the general formula (3) is bound.

The present invention includes a method for disinfecting or inactivating (killing) viruses (particularly, coronaviruses such as the novel coronavirus) using (applying on the site of application) the copolymer (anticoronaviral agent or antiviral agent).

ADVANTAGEOUS EFFECTS OF INVENTION

The anticoronaviral agent comprising the disclosed polymer (amino group-containing copolymer) has antiviral activity against coronaviruses, in particular, SARS-CoV-2. For example, the disclosed polymer (amino group-containing copolymer) alone or as a composition in combination with another compound can be used as an antiviral product.

DESCRIPTION OF EMBODIMENTS

Hereinafter, the present invention will be described in detail.

Combinations of two or more of the individual preferable embodiments of the present disclosure described below are also preferable embodiments of the present disclosure.

<u>Amino group-containing polymer (copolymer)</u>

The disclosed anticoronaviral agent comprises a polymer having at least a specific structural unit (structural unit (a), a structural unit derived from an amino group-containing monomer). Such a structural unit (or a polymer having such a structural unit) is capable of acting on the coronavirus envelope, and thus can efficiently perform an anticoronaviral function. In addition, such a structural unit (or a polymer having such a structural unit) is often relatively safe (e.g., less likely to cause skin irritation or skin sensitization), although it has an amino group or an ammonium base.

The polymer may have, in addition to the structural unit (a), an additional structural unit (a structural unit different from the structural unit (a)). In one preferable embodiment, the anticoronaviral agent typically comprises a copolymer comprising a structural unit derived from an amino group-containing monomer and a specific structural unit (structural unit (b), a structural unit derived from a hydrophobic monomer (sometimes referred to as a hydrophobic unit, etc.)).

The polymer having a combination of the structural unit (a) and such an additional structural unit can perform an even more enhanced anticoronaviral function.

Antimicrobial polymers against bacteria have been known.

Viruses are much smaller in size than bacteria (about 0.1 μm for coronaviruses, compared to 1 to 2 μm for typical bacteria), and their outermost structure is not a membrane but a capsid or envelope structure. Due to these structural differences, antimicrobial polymers are not necessarily considered to have antiviral activity.

Under such circumstances, the disclosed polymer was unexpectedly found to be capable of performing an antiviral, particularly, anticoronaviral function (particularly an excellent anticoronaviral function). The reason for this is not clear, but might be explained by the following consideration. In the disclosed polymer, the structural unit (a) and an additional structural unit are clearly separated from each other so that the additional structural unit (hydrophobic moiety)

easily penetrates coronaviruses (and helps the structural unit (a) to act on and contact with the envelope) in an efficient manner, and at the same time, the structural unit (a) itself performs a strong anticoronaviral function, thus achieving a synergistic and strong anticoronaviral function. Furthermore, appropriate control of the molecular weight range of the polymer will allow the hydrophobic moiety to efficiently penetrate the envelope of microscopic coronaviruses, thereby enabling the polymer to more efficiently perform its function.

The polymer described above may be referred to as the disclosed polymer, the disclosed copolymer, or the like.

Structural unit (a) (structural unit derived from an amino group-containing monomer)

In the present disclosure, the structural unit (a) (structural unit derived from an amino group-containing monomer) is defined as a structural unit of the general formula (1) below, a structural unit that is a salt (e.g., a salt with an acid) of the general formula (1) below, and/or a structural unit of the general formula (2) below.

[Chem. 7]

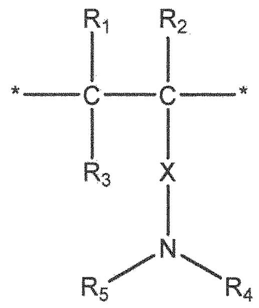

[Chem. 8]

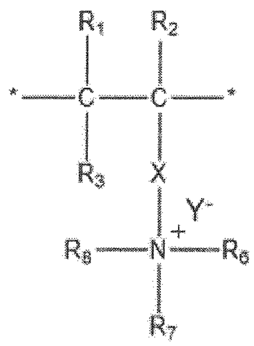

General formula (2)

In the general formula (1) or (2), $R_1$, $R_2$, and $R_3$ each independently represent a hydrogen atom or an alkyl group of 1 to 5 carbon atoms, $R_4$ to $R_8$ each independently represent a hydrogen atom or an organic group (e.g., an organic group of 1 to 12 carbon atoms), X represents a divalent linking group, and $Y^-$ represents an anion.

The asterisk (*) represents an atom contained in another structural unit of the same or different type to which the structural unit represented by the general formula (1), the structural unit that is a salt of the general formula (1), or the structural unit represented by the general formula (2) is bound.

In the present disclosure, the "atom contained in another structural unit of the same type" means, for example, in the case of the structural unit represented by the general formula (1), an atom contained in another structural unit represented by the general formula (1), and the "atom contained in another structural unit of a different type" means, for example, in the case of the structural unit represented by the general formula (1), an atom contained in a structural unit other than that represented by the general formula (1).

The structure derived from an amino group-containing monomer can be formed, for example but not limited to, by radical polymerization of a monomer having an ethylenically unsaturated group and a primary to tertiary amino group represented by the general formula (4) below, or a primary to tertiary amino group neutralized with an acid, or a quaternary ammonium base represented by the general formula (5) below.

[Chem. 9]

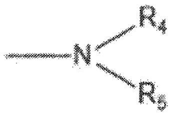

General formula (4)

[Chem. 10]

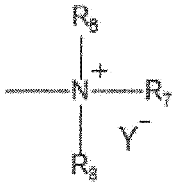

General formula (5)

In the general formulae (4) and (5), $R_4$ to $R_8$ are the same as $R_4$ to $R_8$ in the general formulae (1) and (2).

In the general formula (1) or (2), $R_1$ and $R_2$ are each preferably a hydrogen atom, and $R_3$ is preferably a hydrogen atom or a methyl group.

In the general formulae (1) and (2) (and also (4) and (5)), examples of the organic group include organic groups of 1 to 12 carbon atoms. Examples of the organic group of 1 to 12 carbon atoms include hydrocarbon groups [e.g., aliphatic hydrocarbon groups such as alkyl groups (e.g., $C_{1-12}$ alkyl groups, preferably $C_{1-8}$ alkyl groups, more preferably $C_{1-4}$ alkyl groups, such as a methyl group, an ethyl group, a propyl group, an isopropyl group, an n-butyl group, an s-butyl group, a t-butyl group, etc.) and alkenyl groups (e.g., $C_{2-12}$ alkenyl groups such as a vinyl group, an allyl group, etc.)].

The organic group (hydrocarbon group, etc.) may have a substituent such as a hydroxy group, a halogen atom, etc.

In the formula (1) or (2), $R_4$, $R_5$, $R_6$, $R_7$, and $R_8$ are each preferably a hydrogen atom or a hydrocarbon group (e.g., an alkyl group).

In the formula (1), $R_4$ and $R_5$ may be the same or different, and two of them may be bound to each other or one of them may be bound to X to form a ring with the nitrogen atom (N), or no ring may be formed. Similarly, in the formula (2), $R_6$, $R_7$, and $R_8$ may be the same or different, and two of them may be bound to each other or one of them may be bound to X to form a ring with the nitrogen atom (N), or no ring may be formed.

In the general formula (1) or (2), the divalent linking group X is, for example, a direct bond, an ether group (or an ether bond, -O-), a thioether group (or a thioether bond, -S-), a group containing a carbonyl group (a carbonyl group, an ester group, an amide group, a urethane group, etc.), a hydrocarbon group [e.g., a $C_{1-20}$ alkylene or alkylidene group, preferably a $C_{1-12}$ alkylene or alkylidene group, more preferably a $C_{1-8}$ alkylene or alkylidene group, such as a methylene group, an ethylene group, a trimethylene group, a propylene group, a tetramethylene group, etc.], or a group composed of two or more of the above groups bound together {e.g., a group composed of a hydrocarbon group (such as an alkylene or alkylidene group) bound to another group, such as a group represented by -C(=O)-O-R wherein R represents a hydrocarbon group (e.g., an alkylene group such as an ethylene group or an alkylidene group)}.

The hydrocarbon group may have a substituent such as a hydroxy group or a halogen atom.

Typically, X is not composed simply of an ether group, a thioether group, a carbonyl group, an ester group, an amide group, a urethane group, etc., but is often composed of such a group bound to a hydrocarbon group, etc. in such a manner that such a group is not bound directly to the nitrogen atom (N) of the formula (1) or (2), that is, the hydrocarbon group, etc., is bound to the nitrogen atom (N) of the formula (1) or (2).

X is preferably a group in which a carbonyl group is bound to an alkylene or alkylidene group to form an ester bond (-C(=O)-O-R- wherein R is an alkylene or alkylidene group). More preferably, -X- contains a structural unit represented by - $C(=O)-O-CH_2CH_2-$.

The polymer may have one or more types of structural units (a).

The structural unit derived from an amino group-containing monomer can be formed, for example, by radical polymerization of an amino group-containing monomer.

Specific examples of the amino group-containing monomer include N,N-dialkylamino group-containing (meth)acrylates such as N,N-dimethylaminoethyl (meth)acrylate, N,N-diethylaminoethyl (meth)acrylate, N,N-dimethylaminopropyl (meth)acrylate, and N,N-diethylaminopropyl (meth)acrylate, and these monomers quaternized with a quaternization agent, or these monomers neutralized with an acid such as hydrochloric acid, acetic acid, lactic acid, phosphoric acid, citric acid, succinic acid, malic acid, etidronic acid, ascorbic acid, glutamic acid, or aspartic acid; N,N-dialkylamino group-containing (meth)acrylamides such as N,N-dimethylaminoethyl (meth)acrylamide, N,N-diethylaminoethyl

(meth)acrylamide, N,N-dimethylaminopropyl (meth)acrylamide, and N,N-diethylaminopropyl (meth)acrylamide, and these monomers quaternized with a quaternization agent, or these monomers neutralized with an acid such as hydrochloric acid, acetic acid, lactic acid, phosphoric acid, citric acid, succinic acid, malic acid, etidronic acid, ascorbic acid, glutamic acid, or aspartic acid; monoalkylamino group-containing (meth)acrylates such as monomethylaminoethyl (meth)acrylate, monoethylaminoethyl (meth)acrylate, monomethylaminopropyl (meth)acrylate, monoethylaminopropyl (meth)acrylate, and 2-(tert-butylamino)ethyl (meth)acrylate, and these monomers neutralized with an acid such as hydrochloric acid, acetic acid, lactic acid, phosphoric acid, citric acid, succinic acid, malic acid, etidronic acid, ascorbic acid, glutamic acid, or aspartic acid; monoalkylamino group-containing (meth)acrylamides such as monomethylaminoethyl (meth)acrylamide, monoethylaminoethyl (meth)acrylamide, monomethylaminopropyl (meth)acrylamide, and monoethylaminopropyl (meth)acrylamide, and these monomers neutralized with an acid such as hydrochloric acid, acetic acid, lactic acid, phosphoric acid, citric acid, succinic acid, malic acid, etidronic acid, ascorbic acid, glutamic acid, or aspartic acid; esters of (meth)acrylic acid and alkanolamines, such as 2-aminoethyl (meth)acrylate, and these monomers neutralized with an acid such as hydrochloric acid, acetic acid, lactic acid, phosphoric acid, citric acid, succinic acid, malic acid, etidronic acid, ascorbic acid, glutamic acid, or aspartic acid; N,N-diallylmethylamines and these monomers quaternized with a quaternization agent, or these monomers neutralized with an acid such as hydrochloric acid, acetic acid, lactic acid, phosphoric acid, citric acid, succinic acid, malic acid, etidronic acid, ascorbic acid, glutamic acid, or aspartic acid; allylamines and these monomers neutralized with an acid such as hydrochloric acid, acetic acid, lactic acid, phosphoric acid, citric acid, succinic acid, malic acid, etidronic acid, ascorbic acid, glutamic acid, or aspartic acid; products of an addition reaction of unsaturated monomers having a cyclic ether-containing group of 2 to 8 carbon atoms with amine compounds of 1 to 24 carbon atoms, such as 1-allyloxy-3-dibutylaminopropan-2-ol and 1-allyloxy-3-diethanolaminopropan-2-ol, and these monomers quaternized with a quaternization agent, or these monomers neutralized with an acid such as hydrochloric acid, acetic acid, lactic acid, phosphoric acid, citric acid, succinic acid, malic acid, etidronic acid, ascorbic acid, glutamic acid, or aspartic acid; and alkenyl cyclic amines such as vinylpyrrolidines (1-vinylpyrrolidine, 3-vinylpyrrolidine, etc.), allylpyrrolidines (2-allylpyrrolidine, etc.), and vinylpiperidines (2-vinylpiperidine and 3-vinylpiperidine), and these monomers neutralized with an acid such as hydrochloric acid, acetic acid, lactic acid, phosphoric acid, citric acid, succinic acid, malic acid, etidronic acid, ascorbic acid, glutamic acid, or aspartic acid.

The structural unit derived from an amino group-containing monomer is particularly preferably represented by the general formula (6) below, which is a structural unit derived from an N-substituted aminoalkyl (meth)acrylate such as N,N-dimethylaminoethyl (meth)acrylate.

Therefore, the structural unit (a) may comprise a structural unit derived from an N-substituted aminoalkyl (meth)acrylate, for example, an N-mono- or di-alkylaminoalkyl (meth)acrylate (e.g., N-mono- or di-$C_{1-4}$ alkylamino $C_{1-4}$ alkyl (meth)acrylate) such as N,N-dimethylaminoethyl (meth)acrylate.

[Chem. 11]

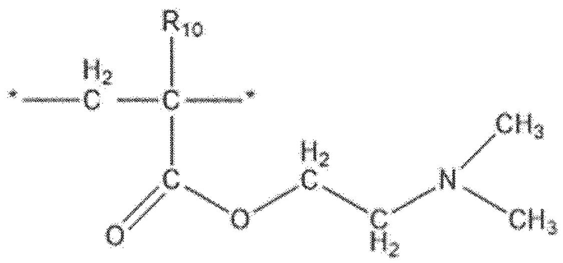

General formula (6)

In the general formula (6), $R_{10}$ represents a hydrogen atom or a methyl group. The asterisk represents an atom contained in another structural unit of the same or different type to which the structural unit represented by the general formula (6) is bound. Most preferably, the structural unit is derived from N,N-dimethylaminoethyl methacrylate.

The amount of the structural unit (a) (structural unit derived from an amino group-containing monomer) in the disclosed polymer (copolymer) may be, for example, 1% by mass or more (e.g., 2% by mass or more, 3% by mass or more, 5% by mass or more, 10% by mass or more, 15% by mass or more, 20% by mass or more, 25% by mass or more, 30% by mass or more, 35% by mass or more, 40% by mass or more, 45% by mass or more, or 50% by mass or more), or 100% by mass or less (e.g., less than 100% by mass, 99% by mass or less, 97% by mass or less, 96% by mass or less, 95% by mass or less, 94% by mass or less, 93% by mass or less, 92% by mass or less, 91% by mass or less, 90% by mass or less, 88% by mass or less, 85% by mass or less, 82% by mass or less, or 80% by mass or less) relative to the total amount of all the structural units (all the monomer-derived structural units constituting the disclosed copolymer), which is assumed as 100% by mass.

These maximum and minimum values may be combined to set an appropriate range for the amount of the structural unit (a). For example, the range may be 1% by mass to 99% by mass, preferably 10% by mass to 97% by

mass, more preferably 36% by mass to 96% by mass, and most preferably 50% by mass to 95% by mass. The above range results in an appropriate hydrophilic-hydrophobic balance and an appropriate ratio of cationic moieties that can act on the surface of the coronavirus and hydrophobic moieties that act on the envelope, as well as potentially helps to maintain the water solubility of the copolymer and thus enhances the anticoronaviral performance of the disclosed anticoronaviral agent.

Structural unit (b) structural unit derived from a monomer containing a hydrocarbon group of 2 or more carbon atoms)

In the present disclosure, the structural unit (b) [structural unit derived from a monomer containing a hydrocarbon group of 2 or more carbon atoms (e.g., in a side chain)] is represented, for example, by the following general formula (3).

[Chem. 12]

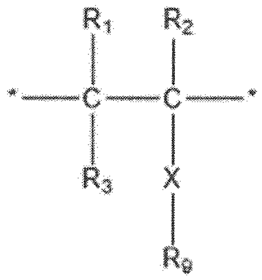

General formula (3)

In the general formula (3), $R_1$, $R_2$, and $R_3$ each independently represent a hydrogen atom or an alkyl group of 1 to 5 carbon atoms, X represents a divalent linking group, and $R_9$ represents a hydrocarbon group of 2 or more carbon atoms optionally having a substituent.

The asterisk represents an atom contained in another structural unit of the same or different type to which the structural unit represented by the general formula (3) is bound.

The structure derived from a monomer containing a hydrocarbon group of 2 or more carbon atoms is, for example, a structural unit in which the carbon-carbon double bond of a monomer having an ethylenically unsaturated group and a hydrocarbon group of 2 or more carbon atoms has been replaced by a carbon-carbon single bond.

The hydrocarbon group of 2 or more carbon atoms may or may not be a radical polymerizable group (e.g., an ethylenically unsaturated group). For example, it may be a saturated hydrocarbon group, an unsaturated hydrocarbon group (such as an alkenyl group), an aromatic hydrocarbon group, etc., and preferably a saturated hydrocarbon group (such as an alkyl group).

The monomer containing a hydrocarbon group of 2 or more carbon atoms has only to have at least one hydrocarbon group of 2 or more carbon atoms, and may have two or more (plural) hydrocarbon groups of 2 or more carbon atoms. When the monomer has two or more carbon hydrocarbon groups of 2 or more carbon atoms, the two or more carbon hydrocarbon groups may be coupled together via an appropriate linking group (e.g., an ether group, a thioether group, an ester group, an amide group, etc.).

$R_9$ may form a ring together with X.

The structural unit derived from a monomer containing a hydrocarbon group of 2 or more carbon atoms can be formed, for example, by radical polymerization of the monomer. Alternatively, the structural unit derived from a monomer containing a hydrocarbon group of 2 or more carbon atoms may be, for example, a structural unit formed by a post-polymerization reaction as long as it is identical to a structural unit in which the carbon-carbon double bond of the monomer has been replaced by a carbon-carbon single bond.

The structural unit of the general formula (3) does not fall under the scope of the structural unit (a). For example, the monomer containing a hydrocarbon group of 2 or more carbon atoms may have a substituent such as a hydroxy group or a halogen atom as long as it contains a hydrocarbon group of 2 or more carbon atoms, but it does not have the group represented by the formula (4), the salt (salt with an acid) of the group represented by the formula (4), or the group represented by the formula (5).

In the general formula (3), the divalent linking group X is, for example, a direct bond, an ether group (or ether bond, -O-), a group containing a carbonyl group (an ester group, an amide group, a urethane group, etc.), or a group composed of any of these groups bound to a hydrocarbon group {e.g., an oxyalkylene group or an oxyalkylidene group [e.g., an oxy $C_{1-20}$ alkylene or alkylidene group such as an oxymethylene group ($-OCH_2-$) or an oxyethylene group]}. X is preferably an ester group or an amide group, and X is more preferably an ester group.

The polymer may have one or more types of structural units (b).

Examples of the monomer containing a hydrocarbon group of 2 or more carbon atoms include, but are not

limited to, esters of (meth)acrylic acid and optionally substituted alcohols (optionally substituted (meth)acrylates); aromatic vinyl monomers such as styrene; esters of unsaturated alcohols and carboxylic acids such as vinyl propionate and vinyl butyrate; alkyl vinyl ethers of 2 or more carbon atoms such as ethyl vinyl ether; products of an addition reaction of unsaturated monomers having a cyclic ether-containing group of 2 to 8 carbon atoms with alcohols of 1 to 20 carbon atoms, such as 1-allyloxy-3-butoxypropan-2-ol; alkylene oxide adducts of unsaturated alcohols of 2 to 20 carbon atoms, such as ethylene oxide adducts of allyl alcohols, ethylene oxide adducts of methallyl alcohols, and ethylene oxide adducts of isoprenol, and their modified products having a hydrophobic terminus; and cyclic vinyl monomers such as N-vinylpyrrolidone.

The monomer containing a hydrocarbon group of 2 or more carbon atoms is preferably a monomer containing at least one type of (meth)acrylic acid ester.

The (meth)acrylic acid ester is preferably an ester of (meth)acrylic acid and a monoalcohol preferably having a hydrocarbon group of 2 to 20 carbon atoms.

More preferably, the monoalcohol has a hydrocarbon group of 2 to 16 carbon atoms, even more preferably 2 to 12 carbon atoms, and particularly preferably 2 to 8 carbon atoms.

When the hydrocarbon group has 2 to 20 carbon atoms, the polymer has an appropriate water solubility and viscosity and is easy to handle. When the hydrocarbon group has 2 to 12 carbon atoms, the polymer is easy to produce and is excellent in terms of safety as well as anticoronaviral activity. When the hydrocarbon group has 2 to 8 carbon atoms, the polymer is not only easy to produce, but also safe, and has an increased affinity with the surface protein of coronaviruses, resulting in an enhanced anticoronaviral activity.

The hydrocarbon group here is preferably an alkyl group (a $C_{2-20}$ alkyl group, a $C_{2-16}$ alkyl group, a $C_{2-12}$ alkyl group, a $C_{2-8}$ alkyl group, etc.) or an alkenyl group, and more preferably an alkyl group.

The structural unit (b) (or hydrophobic unit, e.g., a monomer containing a hydrocarbon group of 2 or more carbon atoms) has a solubility parameter (Hildebrand solubility parameter) [solubility parameter in (as) a homopolymer] of 35 $(MPa)^{1/2}$ or less (e.g., 33 $(MPa)^{1/2}$ or less), preferably 32 $(MPa)^{1/2}$ or less (e.g., 30.8 $(MPa)^{1/2}$ or less), more preferably 30 $(MPa)^{1/2}$ or less (e.g., 28.7 $(MPa)^{1/2}$ or less), even more preferably about 28 $(MPa)^{1/2}$ or less (e.g., 26.7 $(MPa)^{1/2}$ or less). It may also be 25 $(MPa)^{1/2}$ or less (e.g., 24.6 $(MPa)^{1/2}$ or less).

The solubility parameter (minimum value of the solubility parameter) of the structural unit (b) is not particularly limited and may be, for example, 5 $(MPa)^{1/2}$ or more (e.g., 8 $(MPa)^{1/2}$ or more, 10 $(MPa)^{1/2}$ or more, or 10.3 $(MPa)^{1/2}$ or more).

When the polymer has two or more types of structural units (b), at least one type of structural unit (b) may have such a solubility parameter as described above, or all the structural units (b) may have such a solubility parameter as described above.

When the polymer has two or more types of structural units (b), such a solubility parameter as described above may be the value for all the structural units (b) as a whole (average value). In this case, the solubility parameter for all the structural units (b) as a whole can be calculated, for example, using the solubility parameter of each structural unit (b) (or each monomer) and the volume fraction of each structural unit (b) (or each monomer).

The solubility parameter herein means the Hildebrand solubility parameter (SP value), and for example, the value calculated by the method described on pages 147-154 of "POLYMER ENGINEERING AND SCIENCE" (1974, Vol. 14, No. 2) can be used as reference.

The (meth)acrylic acid ester here is preferably a (meth)acrylic acid alkyl ester (alkyl (meth)acrylate).

In particular, the structural unit represented by the general formula (7) below, which is a structural unit derived from ethyl (meth)acrylate, is preferable.

Therefore, the structural unit (b) may comprise a structural unit derived from a (meth)acrylic acid alkyl ester [an alkyl (meth)acrylate such as a $C_{1-4}$ alkyl (meth)acrylate (such as ethyl (meth)acrylate)].

[Chem. 13]

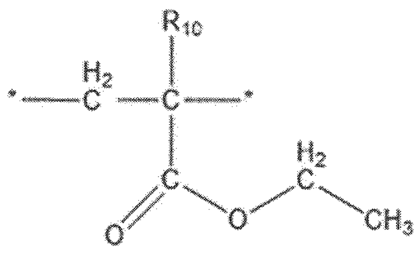

General formula (7)

In the general formula (7), $R_{10}$ represents a hydrogen atom or a methyl group. The asterisk represents an atom

contained in another structural unit of the same or different type to which the structural unit represented by the general formula (7) is bound. Most preferably, the structural unit is derived from ethyl methacrylate.

The amount of the structural unit (b) (structural unit derived from a hydrophobic monomer) in the disclosed polymer (copolymer) may be, for example, 1% by mass or more (e.g., 2% by mass or more, 3% by mass or more, 5% by mass or more, 10% by mass or more, 15% by mass or more, 20% by mass or more, 25% by mass or more, or 30% by mass or more), or 99% by mass or less (e.g., 97% by mass or less, 95% by mass or less, 90% by mass or less, 85% by mass or less, 80% by mass or less, 75% by mass or less, 70% by mass or less, 65% by mass or less, 60% by mass or less, 55% by mass or less, or 50% by mass or less) relative to the total amount of all the structural units (all the monomer-derived structural units constituting the disclosed copolymer), which is assumed as 100% by mass.

These maximum and minimum values may be combined to set an appropriate range for the amount of the structural unit (b). For example, the range is 1 % by mass to 99% by mass, preferably 3% by mass to 90% by mass, more preferably 4% by mass to 70% by mass, and most preferably 5% by mass to 50% by mass.

When the amount is in this range, the polymer (copolymer) has an appropriate hydrophobicity and an enhanced anticoronaviral performance, that is, a higher antiviral performance.

Additional structural unit

The disclosed polymer (copolymer) may, if desired, have an additional structural unit derived from a monomer (also referred to as an additional structural unit derived from some other monomer) other than the structural unit derived from an amino group-containing monomer or the structural unit derived from a monomer containing a hydrocarbon group of 2 or more carbon atoms.

There are no particular limitations on the some other monomer as long as it has no amino group and is capable of copolymerizing with the amino group-containing monomer and the monomer containing a hydrocarbon group of 2 or more carbon atoms. As long as the polymer comprises the structural unit (a) and the structural unit (b) in an appropriate combination and ratio (e.g., a favorable fraction of the monomer containing a hydrocarbon group of 2 or more carbon atoms is polymerized in the polymer), the function (e.g., hydrophobicity) as a copolymer can be sufficiently maintained.

In the present disclosure, the additional structural unit derived from some other monomer is a structural unit in which at least one carbon-carbon double bond of the some other monomer has been replaced by a carbon-carbon single bond. As long as the additional structural unit derived from some other monomer is identical to the structural unit in which at least one carbon-carbon double bond of the some other monomer has been replaced by a carbon-carbon single bond, the additional structural unit is not limited to a structural unit formed by polymerization of the some other monomer and may be, for example, a structural unit formed by a post-polymerization reaction.

Examples of the additional structural unit derived from some other monomer include, but are not limited to, unsaturated monocarboxylic acids such as (meth)acrylic acid, crotonic acid, $\alpha$-allyloxy acrylic acid, and their salts; olefin monomers such as propylene; vinyl halides such as vinyl chloride; and methyl vinyl ether.

The polymer may have one or more types of additional structural units.

The amount of the additional structural unit derived from some other monomer in the disclosed polymer (copolymer) is less than 15% by mass relative to the total amount of all the monomer-derived structural units constituting the disclosed polymer (copolymer), which is assumed as 100% by mass. It is preferably less than 10% by mass, and more preferably less than 5% by mass. One of these monomers may be used alone, and also two or more of them may be used in combination.

Physical properties, etc. of the amino group-containing polymer (copolymer) of the present disclosure

The disclosed polymer (copolymer) preferably has a weight-average molecular weight (Mw) of 4000 to 800000, more preferably 6000 to 400000, and even more preferably 7000 to 80000. Within the above range, the disclosed anticoronaviral agent has an enhanced anticoronaviral performance and is easy to handle due to its excellent water solubility and dispersibility.

The weight-average molecular weight of the disclosed polymer (copolymer) can be measured using known techniques. Specifically, the GPC method and the viscosity method can be used.

The molecular weight distribution index of the disclosed polymer (copolymer) is preferably 1.1 or more, more preferably 1.2 or more, even more preferably 1.3 or more; or preferably 5.0 or less, more preferably 4.0 or less, even more preferably 3.0 or less.

The solubility parameter (Hildebrand solubility parameter) of the disclosed polymer (copolymer) may be, for example, 8 $(MPa)^{1/2}$ or more (e.g., 10 $(MPa)^{1/2}$ or more), preferably 12 $(MPa)^{1/2}$ or more (e.g., 13 $(MPa)^{1/2}$ or more), more preferably 14 $(MPa)^{1/2}$ or more (e.g., 14.5 $(MPa)^{1/2}$ or more), and even more preferably about 15 $(MPa)^{1/2}$ or more.

The solubility parameter (Hildebrand solubility parameter) (maximum value of the solubility parameter) of the disclosed polymer (copolymer) may be, for example, 30 $(MPa)^{1/2}$ or less (e.g., 28 $(MPa)^{1/2}$ or less, 26 $(MPa)^{1/2}$ or less,

25 $(MPa)^{1/2}$ or less, or 24 $(MPa)^{1/2}$ or less), preferably 23 $(MPa)^{1/2}$ or less (e.g., 22 $(MPa)^{1/2}$ or less), more preferably 21 $(MPa)^{1/2}$ or less (e.g., 20.5 $(MPa)^{1/2}$ or less), and even more preferably 20 $(MPa)^{1/2}$ or less.

The δd (dispersion term) in the Hansen solubility parameter of the disclosed polymer (copolymer) is, for example, preferably 10 $(MPa)^{1/2}$ or more, more preferably 11 $(MPa)^{1/2}$ or more, even more preferably 12 $(MPa)^{1/2}$ or more; or preferably 21 $(MPa)^{1/2}$ or less, more preferably 20 $(MPa)^{1/2}$ or less, even more preferably 19 $(MPa)^{1/2}$ or less.

The δp (polar term) in the Hansen solubility parameter of the disclosed polymer (copolymer) is, for example, preferably 3.0 $(MPa)^{1/2}$ or more, more preferably 4.0 $(MPa)^{1/2}$ or more, even more preferably 5.0 $(MPa)^{1/2}$ or more; or preferably 11.0 $(MPa)^{1/2}$ or less, more preferably 10.0 $(MPa)^{1/2}$ or less, even more preferably 9.0 $(MPa)^{1/2}$ or less.

The δh (hydrogen-bonding term) in the Hansen solubility parameter of the disclosed polymer (copolymer) is, for example, preferably 3.0 $(MPa)^{1/2}$ or more, more preferably 4.0 $(MPa)^{1/2}$ or more, even more preferably 5.0 $(MPa)^{1/2}$ or more; or preferably 11.0 $(MPa)^{1/2}$ or less, more preferably 10.0 $(MPa)^{1/2}$ or less, even more preferably 9.0 $(MPa)^{1/2}$ or less.

The Hansen solubility parameter is a three-dimensional solubility parameter derived from the Hildebrand solubility parameter, which is composed of three components: dispersion term δd, polar term δp, and hydrogen-bonding term δh. The dispersion term δd represents the effect of nonpolar interactions, the polar term δρ represents the effect of dipole-dipole interactions, and the hydrogen-bonding term δh represents the effect of hydrogen-bonding forces.

The solubility parameter (Hildebrand solubility parameter or Hansen solubility parameter) of the disclosed polymer (copolymer) can be calculated using the solubility parameter of each monomer used in the polymer (copolymer) [(unit derived from) each monomer constituting the polymer (copolymer)] and the volume fraction of each monomer.

An exemplary calculation is shown below. This example describes the Hansen solubility parameter of a copolymer formed by polymerizing 60 parts by mass of N,N-dimethylaminoethyl methacrylate and 40 parts by mass of ethyl methacrylate.

First, the Hansen solubility parameters for N,N-dimethylaminoethyl methacrylate and ethyl methacrylate are as follows:

N,N-dimethylaminoethyl methacrylate (δd = 14.3 $(MPa)^{1/2}$, δp = 7.0 $(MPa)^{1/2}$, δh = 7.7 $(MPa)^{1/2}$)
ethyl methacrylate (δd = 15.8 $(MPa)^{1/2}$, δp = 7.2 $(MPa)^{1/2}$, δh = 7.5 $(MPa)^{1/2}$).

Assuming that the specific gravity of N,N-dimethylaminoethyl methacrylate is 0.9 and that of ethyl methacrylate is 0.9, the volume ratio of N,N-dimethylaminoethyl methacrylate:ethyl methacrylate is 60:40.

Accordingly, the Hansen solubility parameter of the copolymer consisting of 60 parts by mass of N,N-dimethylaminoethyl methacrylate and 40 parts by mass of ethyl methacrylate can be calculated as follows.

The δd of the copolymer = $14.3\times0.6+15.8\times0.4=14.9\ (MPa)^{1/2}$
The δp of the copolymer = $7.0\times0.6+7.2\times0.4=7.1\ (MPa)^{1/2}$
The δh of the copolymer = $7.7\times0.6+7.5\times0.4=7.6\ (MPa)^{1/2}$

The values of the Hansen solubility parameter for each monomer used in the above calculation are taken from Hansen Solubility Parameters: A User's Handbook.

The solubility parameter as described above is preferable because it facilitates efficient anticoronaviral function.

The disclosed copolymer may be a random copolymer, a block copolymer, a graft copolymer, etc.

Method for producing the amino group-containing polymer (copolymer) of the present disclosure

The method for producing the disclosed polymer (copolymer) is not particularly limited, but usually preferred is the polymerization of at least the monomer as the origin of the structural unit (a) (e.g., an amino group-containing monomer and a monomer containing a hydrocarbon group of 2 or more carbon atoms, and optionally some other monomer). The polymer (copolymer) can be produced by, for example but not limited to, solution polymerization, bulk polymerization, suspension polymerization, emulsion polymerization, living polymerization, or graft polymerization, and preferred is solution polymerization.

The solvent used in this process is not particularly limited, and preferable examples of the solvent include water; monovalent alcohols such as methyl alcohol, ethyl alcohol, isopropyl alcohol, butanol, and THF (tetrahydrofuran); polyhydric alcohols such as glycerin, (poly)ethylene glycol, propylene glycol, 1,3-butylene glycol, and dipropylene glycol; aromatic or aliphatic hydrocarbons such as benzene, toluene, xylene, cyclohexane, and n-heptane; esters such as ethyl acetate; ketones such as acetone and methyl ethyl ketone; amides such as dimethyl formamide; and ethers such as diethyl ether and dioxane. One of these solvents may be used alone, and also two or more of them may be used in combination.

Among these, one or more solvents selected from the group consisting of water and lower alcohols of 1 to 4

carbon atoms are preferable in terms of the solubility of the monomer component and the resultant polymer (copolymer). A polyhydric alcohol solvent such as propylene glycol or ethylene glycol may be mixed with water and used as a solvent for polymerization.

Combining such a polyhydric alcohol solvent with water can increase the solubility of the polymer and more sufficiently inhibit soap-free polymerization. This can more sufficiently inhibit the formation of poorly water-soluble polymers and further improve the transparency of the solution. Using water, ethyl alcohol, glycerin, 1,3-butanediol, or a combination of two or more of them as a solvent is more preferable in that they can be used directly as ingredients for skin topical agents without the need for a solvent removal process of the polymer product.

A neutralized product of the disclosed polymer (copolymer) can be produced by polymerizing a monomer that has been neutralized, as described in the section on the structural unit derived from an amino group-containing monomer, or by polymerizing a monomer that has not been neutralized and then neutralizing the resulting polymer with a given acid.

Coronavirus

A coronavirus is an enveloped virus with a positive-sense single-stranded RNA genome and a nucleocapsid of helical symmetry. The coronaviruses according to the present disclosure include coronaviruses that infect humans or non-human animals.

The coronaviruses that infect humans include human coronaviruses 229E, OC43, NL63, and HKU-1, severe acute respiratory syndrome (SARS) coronavirus, Middle East respiratory syndrome (MERS) coronavirus, and the novel coronavirus (2019-nCoV, SARS-CoV-2).

The coronaviruses that infect non-human animals include porcine epidemic diarrhea virus (PEDV), porcine transmissible gastroenteritis virus (TGEV), feline infectious peritonitis virus (FIPV), canine coronavirus (CCV), bovine coronavirus, equine coronavirus, chicken infectious bronchitis virus (IBV), avian delta coronavirus, and porcine delta coronavirus.

The coronavirus according to the present disclosure may be SARS-CoV-2, which is a causative virus of the novel coronavirus infection (COVID-19) and belongs to the family *Coronaviridae.* So-called SARS-CoV-2 variants (e.g., those with spike protein mutations such as N501Y, L452R, E484Q, D614G, P681H, Y145H, A222V, and E484K) are also included in the scope of the coronavirus according to the present disclosure.

The currently known (publicly known) SARS-CoV-2 variants include so-called alpha, beta, gamma, delta, kappa, lambda, mu, and omicron variants. The target of the disclosed anticoronaviral agent includes not only these known variants but also currently unknown variants and new variants that may emerge as a result of future mutations.

The target of the disclosed anticoronaviral agent is not particularly limited. The anticoronaviral agent can be used for the purpose of inhibiting coronavirus infection in livestock and pets, or for the purpose of inhibiting human coronavirus infection due to its antiviral activity against coronaviruses that infect humans, such as SARS coronavirus, MERS coronavirus, SARS-CoV-2, and in recent years, particularly SARS-CoV-2.

Anticoronaviral agent

The disclosed anticoronaviral agent (composition, anticoronaviral composition) comprises the disclosed polymer (copolymer) as an essential ingredient. The maximum amount of the disclosed polymer (copolymer) in the disclosed anticoronaviral agent may be 100% by mass. The minimum amount is not particularly limited, but the amount of the disclosed polymer is preferably 0.01% by mass or more to ensure the effectiveness of the formulation.

The disclosed anticoronaviral agent may further comprise a solvent such as water, an antimicrobial agent, or a preservative. Other ingredients are not particularly limited as long as they do not interfere with the anticoronaviral performance of the anticoronaviral agent. Examples include alkaline regulators, anionic surfactants, compatibilizers, stabilizers, antimicrobial agents, and other additives.

The anticoronaviral agent of the present invention may further comprise a metal salt, a metal oxide, or a metal hydroxide for the purpose of enhancing its anticoronaviral activity. The amount of the above other ingredients is not particularly limited as long as they do not interfere with the anticoronaviral performance of the anticoronaviral agent, but is preferably 0 to 20% by mass relative to the amount of the polymer (copolymer), which is assumed as 100% by mass.

The anticoronaviral agent is typically in a form containing the disclosed polymer (copolymer) and a solvent [e.g., a solution (particularly an aqueous solution)].

Examples of the solvent include water and aqueous solvents [e.g., alcohols (e.g., methanol, ethanol, isopropanol, etc.), etc.], and optionally mixed solvents.

In such a form (e.g., a solution), the concentration of the polymer (copolymer) may be, for example, 0.001% by mass or more, preferably 0.005% by mass or more, and more preferably about 0.01% by mass or more.

The anticoronaviral agent can be used after diluted as appropriate. The maximum concentration is not particularly limited, and the concentration may be, for example, 90% by mass or less, 80% by mass or less, 50% by mass or

less, 30% by mass or less, 20% by mass or less, 10% by mass or less, 5% by mass or less, 3% by mass or less, 2% by mass or less, or 1% by mass or less.

In the form containing a solvent (water), the pH (e.g., pH at 25°C) is not particularly limited and may be in an acidic, neutral, or alkaline range. For example, a (weakly) acidic to (weakly) alkaline range, such as 3 to 11, 4 to 10, 5 to 9, 6 to 8, 3 to 8, 3 to 7, 4 to 8, 4.5 to 7.5, 5 to 7, or 4 to 6.5, may be selected according to the application, the application site, the subject to be treated, etc.

The pH may be, for example, 11 or less, 10 or less, or 9 or less. Such a pH is preferable in that the polymer is sufficiently soluble in water, and a smaller amount of the polymer can effectively act in an aqueous solution. Referring to the minimum value, the pH may be 2 or more, 3 or more, or 4 or more. Such a pH is preferable because it provides an appropriate degree of cationization and allows the hydrophobic moiety to efficiently act on the envelope. Moreover, such a pH is preferable in terms of stability during the formulation process and irritation upon exposure to the living body.

The anticoronaviral agent of the present invention can optionally comprise additives commonly used in pharmaceuticals, quasi-drugs, cosmetics, laundry detergents, general goods such as packs and sheet masks, and hygiene products. Examples of the additive include oil bases, moisturizers, feel enhancers, surfactants, polymers, thickeners/gellants, solvents, propellants, antioxidants, reductants, oxidants, antiseptic/antimicrobial agents other than antiseptic/antimicrobial agents of the present invention, chelators, pH regulators, acids, alkalis, powders, inorganic salts, UV absorbers, whitening agents, vitamins and their derivatives, antiphlogistics, antiinflammatory agents, hair growers, blood circulation promoters, stimulants, hormones, anti-wrinkle agents, anti-aging agents, astringents, cooling sensation agents, warming sensation agents, wound healing promoters, irritation relieving agents, analgesics, cell activators, plant/animal/microbial extracts, antipruritics, peeling/ keratolytic agents, antiperspirants, fresheners, astringents, enzymes, nucleic acids, fragrances, colorings, colorants, dyes, pigments, and various types of water.

The disclosed anticoronaviral agent may be used in combination with silver ions and/or polyalkylenimines (including their derivatives).

## Applications of the anticoronaviral agent

The disclosed anticoronaviral agent can be used as an additive for the purpose of imparting antiviral activity. For example, the anticoronaviral agent can be used in pharmaceuticals, quasi-drugs, cosmetics, general goods such as packs and sheet masks, laundry detergents, hygiene products, etc. When used for these applications, the anticoronaviral agent can be formulated according to the method appropriate for each application. For example, the anticoronaviral agent can be formulated into a solution in a variety of solvents, an emulsion, a gel, a foam, or a spray, or a sheet impregnated therewith.

The disclosed anticoronaviral agent may also be applied to a substrate to form an anticoronaviral film to produce a laminated product having an anticoronaviral effect. The type of the substrate is not particularly limited as long as the substrate serves to support the anticoronaviral agent. The substrate may be a part of a variety of devices (e.g., a front plate).

The form of the substrate is not particularly limited, and examples include plates, films, sheets, tubes, fibers, and particles. The geometry of the substrate surface on which the anticoronaviral film is placed is not particularly limited and may be flat, concave, convex, or a combination thereof.

The material of the substrate is not particularly limited, and examples include wood, metal, glass, ceramics, and plastics (resins). In particular, plastics are preferable in terms of ease of handling. That is, a resin substrate is preferable.

Exemplary methods for applying the anticoronaviral agent to the surface of the substrate include spray coating, wire bar coating, extrusion coating, direct gravure coating, reverse gravure coating, ink-jet coating, and die coating.

## Usage of the anticoronaviral agent

The method for inhibiting coronavirus growth or inhibiting coronaviruses using the disclosed anticoronaviral agent is a preferable embodiment. Particularly preferably, the disclosed anticoronaviral agent is for use against SARS-CoV-2.

The antiviral activity value of the disclosed anticoronaviral agent is preferably 1 or more (particularly >1), more preferably 1.5 or more, even more preferably 2 or more, and may even be 3 or more.

The antiviral activity value according to the present disclosure can be calculated by the equation below using the infectious titer of the virus determined by the plaque assay.

Equation:

Antiviral activity value = Log (infectious titer of the virus after exposure to the control) - Log (infectious titer of the virus after exposure to the anticoronaviral agent)

The control can be selected as appropriate and may be water, saline, a buffer (e.g., PBS), etc.

The antiviral activity value as described above may be achieved in a relatively short time (e.g., within 10, 20, or 30 minutes).

On the other hand, the antiviral activity value as described above may be maintained over a long period of time (e.g., 1 hour or more, 1.5 hours or more, 2 hours or more, 6 hours or more, 12 hours or more, 1 day or more, 3 days or more, 7 days or more, 10 days or more, etc.).

EXAMPLES

Hereinafter, the present invention will be described in more detail by examples, but the present invention is not limited thereto. In the following, the unit "part" refers to part by mass, and the sign "%" refers to percent by mass unless otherwise specified.

Method for measurement of weight-average molecular weight

The weight-average molecular weight (Mw) of copolymers was measured by GPC (gel permeation chromatography). The measurement conditions and apparatus are as follows.

Instrument: e2695 from Waters
Detector: refractive index (RI) detector
Columns: TSKgel α-M and α-2500 from Tosoh Bioscience
Column temperature: 40°C
Flow rate: 0.8 mL/min
Injection volume: 10 μL (0.4 wt% sample solution in eluent)
Calibration curve: polyethylene glycol from GL Sciences
GPC software: EMPOWERS from Waters
Eluent: 0.5M acetic acid + 0.2M sodium nitrate/acetonitrile = 50/50 (v/v)

Method for measurement of solid content of polymers

About 1 g of the resulting polymer solution was weighed and dried in a hot-air dryer at 200°C for 15 minutes. The solid content of the polymer was expressed as the mass of the residue after drying in percentage relative to the mass before drying.

Method for measurement of pH

The pH value was measured at 25°C with a pH meter (F-72 manufactured by Horiba).

Production Example 1

Into a glass separable flask equipped with a thermometer, a reflux condenser, and a stirrer, 11.1 g of pure water and 100.0 g of 1,3-butanediol (manufactured by Daicel Corporation) were fed. The temperature was raised to 90°C with stirring.

Then, a monomer solution 1 consisting of 60.0 g of 2-(dimethylamino)ethyl methacrylate (also known as N,N-dimethylaminoethyl methacrylate) (referred to as DAM, manufactured by Kyoeisha Chemical Co., LTD.), a monomer solution 2 consisting of 40.0 g of ethyl methacrylate (referred to as EMA, manufactured by Kyoeisha Chemical Co., LTD.), and an aqueous initiator solution consisting of 36.6 g of a 3% aqueous solution of 2,2'-azobis(2-methylpropionamidine) dihydrochloride (referred to as V-50, manufactured by FUJIFILM Wako Pure Chemical Corporation) were added dropwise through separate drop nozzles into a polymerization reaction system at a constant temperature of 90°C with stirring.

The monomer solutions 1 and 2 and the aqueous initiator solution were started to be added simultaneously. The monomer solution 1 was added dropwise for 180 minutes, the monomer solution 2 was added dropwise for 170

minutes, and the aqueous initiator solution was added dropwise for 210 minutes. After the addition was completely finished, the reaction mixture was kept at 90°C for another 30 minutes for aging. After the polymerization was completed, 52.5 g of pure water and 200.0 g of 1,3-butanediol were added to yield a copolymer 1.

The obtained copolymer had a solid content of 19.8%, a pH of 9.0, and a weight-average molecular weight of 48,000. Pure water and citric acid monohydrate were added to the copolymer 1, and the mixture was stirred to give a 2% aqueous copolymer solution having a pH of 6.

Production Example 2

Into a glass separable flask equipped with a thermometer, a reflux condenser, and a stirrer, 11.1 g of pure water and 100.0 g of 1,3-butanediol (manufactured by Daicel Corporation) were fed. The temperature was raised to 90°C with stirring.

Then, a monomer solution 1 consisting of 60.0 g of 2-(dimethylamino)ethyl methacrylate (also known as N,N-dimethylaminoethyl methacrylate) (referred to as DAM, manufactured by Kyoeisha Chemical Co., LTD.), a monomer solution 2 consisting of 40.0 g of ethyl methacrylate (referred to as EMA, manufactured by Kyoeisha Chemical Co., LTD.), and an aqueous initiator solution consisting of 27.9 g of a 10% aqueous solution of 2,2'-azobis(2-methylpropionamidine) dihydrochloride (referred to as V-50, manufactured by FUJIFILM Wako Pure Chemical Corporation) were added dropwise through separate drop nozzles into a polymerization reaction system at a constant temperature of 90°C with stirring.

The monomer solutions 1 and 2 and the aqueous initiator solution were started to be added simultaneously. The monomer solution 1 was added dropwise for 180 minutes, the monomer solution 2 was added dropwise for 170 minutes, and the aqueous initiator solution was added dropwise for 210 minutes. After the addition was completely finished, the reaction mixture was kept at 90°C for another 30 minutes for aging. After the polymerization was completed, 61.1 g of pure water and 200.0 g of 1,3-butanediol were added to yield a copolymer 2.

The obtained copolymer had a solid content of 19.8%, a pH of 9.1, and a weight-average molecular weight of 20,000. Pure water and citric acid monohydrate were added to the copolymer 2, and the mixture was stirred to give a 2% aqueous copolymer solution having a pH of 6.

Production Example 3

Into a glass separable flask equipped with a thermometer, a reflux condenser, and a stirrer, 104.1 g of pure water was fed. The temperature was raised to 90°C with stirring.

Then, a monomer solution 1 consisting of 90.0 g of 2-(dimethylamino)ethyl methacrylate (also known as N,N-dimethylaminoethyl methacrylate) (referred to as DAM, manufactured by Kyoeisha Chemical Co., LTD.), a monomer solution 2 consisting of 10.0 g of ethyl methacrylate (referred to as EMA, manufactured by Kyoeisha Chemical Co., LTD.), 32.7 g of acetic acid (manufactured by FUJIFILM Wako Pure Chemical Corporation), and an aqueous initiator solution consisting of 26.4 g of a 2.5% aqueous solution of 2,2'-azobis(2-methylpropionamidine) dihydrochloride (referred to as V-50, manufactured by FUJIFILM Wako Pure Chemical Corporation) were added dropwise through separate drop nozzles into a polymerization reaction system at a constant temperature of 90°C with stirring.

The monomer solutions 1 and 2, the acetic acid, and the aqueous initiator solution were started to be added simultaneously. The monomer solution 1 was added dropwise for 180 minutes, the monomer solution 2 was added dropwise for 120 minutes, the acetic acid was added dropwise for 180 minutes, and the aqueous initiator solution was added dropwise for 210 minutes. After the addition was completely finished, the reaction mixture was kept at 90°C for another 30 minutes for aging. After the polymerization was completed, 236.7 g of pure water was added to yield a copolymer 3.

The obtained copolymer had a solid content of 20.0%, a pH of 6.4, and a weight-average molecular weight of 58,000. Pure water and citric acid monohydrate were added to the copolymer 3, and the mixture was stirred to give a 2% aqueous copolymer solution.

Production Example 4

Into a glass separable flask equipped with a thermometer, a reflux condenser, and a stirrer, 100.1 g of pure water was fed. The temperature was raised to 90°C with stirring.

Then, a monomer solution 1 consisting of 90.0 g of 2-(dimethylamino)ethyl methacrylate (also known as N,N-dimethylaminoethyl methacrylate) (referred to as DAM, manufactured by Kyoeisha Chemical Co., LTD.), a monomer solution 2 consisting of 10.0 g of ethyl methacrylate (referred to as EMA, manufactured by Kyoeisha Chemical Co., LTD.), 32.7 g of acetic acid (manufactured by FUJIFILM Wako Pure Chemical Corporation), and an aqueous initiator solution consisting of 42.4 g of a 5.0% aqueous solution of 2,2'-azobis(2-methylpropionamidine) dihydrochloride (referred

to as V-50, manufactured by FUJIFILM Wako Pure Chemical Corporation) were added dropwise through separate drop nozzles into a polymerization reaction system at a constant temperature of 90°C with stirring.

The monomer solutions 1 and 2, the acetic acid, and the aqueous initiator solution were started to be added simultaneously. The monomer solution 1 was added dropwise for 180 minutes, the monomer solution 2 was added dropwise for 180 minutes, the acetic acid was added dropwise for 180 minutes, and the aqueous initiator solution was added dropwise for 210 minutes. After the addition was completely finished, the reaction mixture was kept at 90°C for another 30 minutes for aging. After the polymerization was completed, 214.2 g of pure water was added to yield a copolymer 4.

The obtained copolymer had a solid content of 20.4%, a pH of 5.7, and a weight-average molecular weight of 18,000. Pure water and citric acid monohydrate were added to the copolymer 4, and the mixture was stirred to give a 2% aqueous copolymer solution.

Production Example 5

Into a glass separable flask equipped with a thermometer, a reflux condenser, and a stirrer, 33.3 g of pure water and 77.8 g of 1,3-butanediol (manufactured by Daicel Corporation) were fed. The temperature was raised to 90°C with stirring.

Then, a monomer solution 1 consisting of 80.0 g of 2-(dimethylamino)ethyl methacrylate (also known as N,N-dimethylaminoethyl methacrylate) (referred to as DAM, manufactured by Kyoeisha Chemical Co., LTD.), a monomer solution 2 consisting of 20.0 g of ethyl methacrylate (referred to as EMA, manufactured by Kyoeisha Chemical Co., LTD.), and an aqueous initiator solution consisting of 34.2 g of a 3% aqueous solution of 2,2'-azobis(2-methylpropionamidine) dihydrochloride (referred to as V-50, manufactured by FUJIFILM Wako Pure Chemical Corporation) were added dropwise through separate drop nozzles into a polymerization reaction system at a constant temperature of 90°C with stirring.

The monomer solutions 1 and 2 and the aqueous initiator solution were started to be added simultaneously. The monomer solution 1 was added dropwise for 180 minutes, the monomer solution 2 was added dropwise for 180 minutes, and the aqueous initiator solution was added dropwise for 210 minutes. After the addition was completely finished, the reaction mixture was kept at 90°C for another 30 minutes for aging. After the polymerization was completed, 66.7 g of pure water and 66.7 g of 1,3-butanediol were added to yield a copolymer 5.

The obtained copolymer had a solid content of 26.6%, a pH of 9.5, and a weight-average molecular weight of 52,000. Pure water and citric acid monohydrate were added to the copolymer 5, and the mixture was stirred to give a 2% aqueous copolymer solution having a pH of 6.

Production Example 6

Into a glass separable flask equipped with a thermometer, a reflux condenser, and a stirrer, 33.3 g of pure water and 77.8 g of 1,3-butanediol (manufactured by Daicel Corporation) were fed. The temperature was raised to 90°C with stirring.

Then, a monomer solution 1 consisting of 80.0 g of 2-(dimethylamino)ethyl methacrylate (also known as N,N-dimethylaminoethyl methacrylate) (referred to as DAM, manufactured by Kyoeisha Chemical Co., LTD.), a monomer solution 2 consisting of 20.0 g of ethyl methacrylate (referred to as EMA, manufactured by Kyoeisha Chemical Co., LTD.), and an aqueous initiator solution consisting of 37.7 g of a 10% aqueous solution of 2,2'-azobis(2-methylpropionamidine) dihydrochloride (referred to as V-50, manufactured by FUJIFILM Wako Pure Chemical Corporation) were added dropwise through separate drop nozzles into a polymerization reaction system at a constant temperature of 90°C with stirring.

The monomer solutions 1 and 2 and the aqueous initiator solution were started to be added simultaneously. The monomer solution 1 was added dropwise for 180 minutes, the monomer solution 2 was added dropwise for 180 minutes, and the aqueous initiator solution was added dropwise for 210 minutes. After the addition was completely finished, the reaction mixture was kept at 90°C for another 30 minutes for aging. After the polymerization was completed, 66.7 g of pure water and 66.7 g of 1,3-butanediol were added to yield a copolymer 6.

The obtained copolymer had a solid content of 26.3%, a pH of 9.2, and a weight-average molecular weight of 15,000. Pure water and citric acid monohydrate were added to the copolymer 6, and the mixture was stirred to give a 2% aqueous copolymer solution having a pH of 6.

The composition and solubility parameters [Hildebrand solubility parameter (SP value), Hansen solubility parameter (HSP values, $\delta p$, $\delta h$, $\delta$)] of the copolymers obtained in Production Examples 1 to 6 are shown in the table below.

The values of the Hansen solubility parameter for each monomer were taken from Hansen Solubility Parameters: A User's Handbook.

[Table 1]

| | Copolymer | | | | | |
|---|---|---|---|---|---|---|
| | 1 | 2 | 3 | 4 | 5 | 6 |
| DMA(pbm) | 60 | 60 | 90 | 90 | 80 | 80 |
| EMA(pbm) | 40 | 40 | 10 | 10 | 20 | 20 |
| SP value[$(MPa)^{1/2}$] | 18.2 | 18.2 | 17.8 | 17.8 | 17.9 | 17.9 |
| $\delta d[(MPa)^{1/2}]$ | 14.9 | 14.9 | 14.5 | 14.5 | 14.6 | 14.6 |
| $\delta p[(MPa)^{1/2}]$ | 7.1 | 7.1 | 7.0 | 7.0 | 7.0 | 7.0 |
| $\delta h[(MPa)^{1/2}]$ | 7.6 | 7.6 | 7.7 | 7.7 | 7.7 | 7.7 |
| pbm: parts by mass | | | | | | |

Anticoronaviral test 1

Antiviral testing was performed in accordance with ASTM E1052 - Standard Practice to Assess the Activity of Bactericides against Viruses in Suspension. The test viral solution used was prepared from a purified form of the novel coronavirus (SARS-CoV-2).

Example 1

0.1 ml of the test viral solution was mixed with 0.9 ml of a test solution, and the mixture was shaken gently at room temperature for a certain period of time. Then, 9.0 ml of SCDLP medium was added to make a 10-fold dilution series, which was added to host Vero E6 cells (ATCC CCL-1586). The cells were cultured for 3 days.

The infectious titer of the virus after exposure was determined by the plaque assay. A 2% aqueous solution of the copolymer obtained in Production Example 1 was diluted with water such that the polymer (copolymer) concentration was 0.1% by mass. The diluted solution was used as the test solution, while PBS (phosphate buffered saline) was used as the control.

PBS was prepared as follows: 8 g of sodium chloride, 0.2 g of potassium chloride, 1.44 g of disodium hydrogen phosphate, and 0.24 g of potassium dihydrogen phosphate were dissolved in water, adjusted to pH 7.4, and then made up to 1 L (the same applies hereinafter).

The antiviral activity value was calculated by the following formula.

Antiviral activity value = Log (infectious titer of the virus after exposure to the control) - Log (infectious titer of the virus after exposure to the test solution of Example 1)

The infectious titer of the virus and the antiviral activity value for the test solution of Example 1 are shown in Table 2.

[Table 2]

| Test solution | | Infectious titer of the virus (pfu/ml) | | | | Antiviral activity value | | |
|---|---|---|---|---|---|---|---|---|
| Type | Copolymer | 0 min | 30 min | 1 h | 2 h | 30 min | 1 h | 2 h |
| Example 1 | Copolymer 1 | 9.50E+06 | 6.75E+05 | 1.00E+05 | 6.50E+03 | 1.23 | 2.0 | 3.21 |
| Control | - | 9.50E+06 | 1.15E+07 | 1.00E+07 | 1.05E+07 | - | - | - |
| * "E+a" in Table 2 means "$\times 10^a$". For example, 9.50E+06 means $9.50\times 10^6$ (the same applies in the corresponding descriptions below). | | | | | | | | |

Example 1, which is an embodiment of the present invention, had an antiviral activity value of 2.0 at 1 hour

post exposure, indicating that it has a sufficiently anticoronaviral activity.

In addition, the disclosed anticoronaviral agent showed an increase in antiviral activity value over time, indicating that it has a sustained anticoronaviral effect.

Anticoronaviral test 2

Antiviral testing was performed in accordance with ASTM E1052 - Standard Practice to Assess the Activity of Bactericides against Viruses in Suspension. The test viral solution used was prepared from porcine epidemic diarrhea (PED) coronavirus.

Examples 2 to 7

1 ml of the test viral solution was mixed with 10 ml of a test solution, and the mixture was shaken gently at room temperature for a certain period of time. Then, after 10-fold dilution in SCDLP medium, a 10-fold dilution series was prepared and added to host Vero cells (ATCC CCL-81). The cells were cultured for 5 days.

The infectious titer of the virus after exposure was determined by the TCID50 assay. A 2% aqueous solution of each copolymer obtained in Production Examples 1 to 6 was diluted with water such that the polymer (copolymer) concentration was 0.1% by mass. Each diluted solution was used as the test solution (test solutions of Examples 2 to 7), while PBS (phosphate buffered saline) was used as the control.

The antiviral activity value was calculated by the following formula.

Antiviral activity value = Log (infectious titer of the virus after exposure to the control) - Log (infectious titer of the virus after exposure to any of the test solutions of Examples 2 to 7)

The infectious titer of the virus and the antiviral activity value for any of the test solutions of Examples 2 to 7 are shown in Table 3.

[Table 3]

| Test solution | | Infectious titer of the virus (pfu/ml) | | Antiviral activity value |
|---|---|---|---|---|
| Type | Copolymer | 0 min | 2 h | 2 h |
| Example 2 | Copolymer 1 | 3.16E+06 | 2.00E+03 | 2.8 |
| Example 3 | Copolymer 2 | 3.16E+06 | 3.16E+04 | 1.6 |
| Example 4 | Copolymer 3 | 3.16E+06 | 1.26E+04 | 2.0 |
| Example 5 | Copolymer 4 | 3.16E+06 | 1.26E+04 | 2.0 |
| Example 6 | Copolymer 5 | 3.16E+06 | 7.94E+03 | 2.2 |
| Example 7 | Copolymer 6 | 3.16E+06 | 3.16E+04 | 1.6 |
| Control | - | 3.16E+06 | 1.26E+06 | - |

Examples 2 to 7, which are embodiments of the present invention, all had an antiviral activity value of 1.6 or more at 2 hours post-exposure, indicating that they have anticoronaviral activity regardless of their molecular weight or composition ratio.

As described above, all of the copolymers used in Examples 1 to 7 have anticoronaviral activity and also have advantages in water solubility, compatibility (ease of blending and dispersibility) with various other ingredients (cosmetics and cosmetic ingredients), and moisturizing effect on the skin. In addition, when any of the copolymers is applied to a substrate of interest, a coating film therefrom has an excellent water resistance and stain resistance.

## Claims

**1.** An anticoronaviral agent comprising a polymer comprising at least one type of structural unit (a) selected from a

structural unit represented by the following general formula (1):

[Chem. 1]

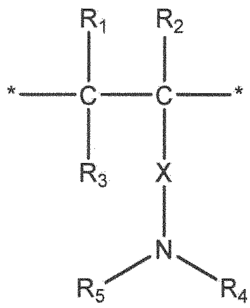

,

a structural unit that is a salt of the general formula (1), and a structural unit represented by the following general formula (2):

[Chem. 2]

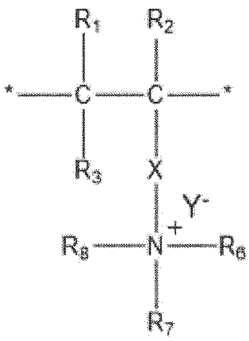

General formula (2)

wherein in the general formulae (1) and (2), $R_1$, $R_2$, and $R_3$ each independently represent a hydrogen atom or an alkyl group of 1 to 5 carbon atoms, $R_4$ to $R_8$ each independently represent a hydrogen atom or an organic group, X represents a divalent linking group, and $Y^-$ represents an anion.

**2.** The anticoronaviral agent according to claim 1, wherein the polymer further comprises a structural unit (b) having a Hildebrand solubility parameter of 35 $(MPa)^{1/2}$ or less.

**3.** The anticoronaviral agent according to claim 1 or 2, wherein the polymer further comprises a structural unit (b) represented by the following general formula (3):

[Chem. 3]

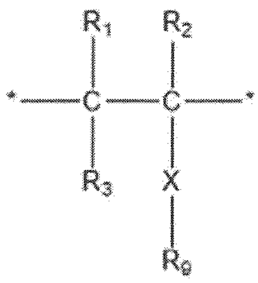

General formula (3)

,

and wherein in the general formula (3), $R_1$, $R_2$, and $R_3$ each independently represent a hydrogen atom or an alkyl group of 1 to 5 carbon atoms, X represents a divalent linking group, and $R_9$ represents a hydrocarbon group of 2 or more carbon atoms optionally having a substituent.

4. The agent according to any one of claims 1 to 3, wherein the polymer has a Hildebrand solubility parameter of 8 to 30 $(MPa)^{1/2}$.

5. The agent according to any one of claims 1 to 4, wherein the polymer further comprises a structural unit (b) having a Hildebrand solubility parameter of 32 $(MPa)^{1/2}$ or less, and wherein the polymer has a Hildebrand solubility parameter of 10 to 25 $(MPa)^{1/2}$.

6. The agent according to any one of claims 2 to 5, wherein the structural unit (a) comprises a structural unit derived from an N-mono- or di-alkylaminoalkyl (meth)acrylate, and wherein the structural unit (b) comprises a structural unit derived from a (meth)acrylic acid alkyl ester.

7. The agent according to any one of claims 1 to 6, wherein the amount of the structural unit (a) in the polymer is 30% by mass or more and the amount of the structural unit (b) in the polymer is 1% by mass or more relative to the total amount of all the structural units, which is assumed as 100% by mass.

8. The agent according to any one of claims 1 to 7, wherein the amount of the structural unit (a) in the polymer is 50% by mass or more and the amount of the structural unit (b) in the polymer is 5% by mass or more relative to the total amount of all the structural units, which is assumed as 100% by mass, and wherein the polymer has a Hildebrand solubility parameter of 12 to 23 $(MPa)^{1/2}$.

9. The agent according to any one of claims 1 to 8, wherein the polymer is present at a concentration of 0.01% by mass or more.

10. A method for inactivating a coronavirus and/or inhibiting coronavirus growth using the agent according to any one of claims 1 to 9.

**INTERNATIONAL SEARCH REPORT**

International application No. **PCT/JP2022/008180**

**A. CLASSIFICATION OF SUBJECT MATTER**

***A61K 31/78***(2006.01)i; ***A01N 61/00***(2006.01)i; ***A01P 1/00***(2006.01)i; ***A61P 31/14***(2006.01)i; ***C08F 220/34***(2006.01)i
FI: A61K31/78; C08F220/34; A61P31/14; A01N61/00 D; A01P1/00

According to International Patent Classification (IPC) or to both national classification and IPC

**B. FIELDS SEARCHED**

Minimum documentation searched (classification system followed by classification symbols)

A61K31/78; A01N61/00; A01P1/00; A61P31/14; C08F220/34

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

CAplus/REGISTRY/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X | US 2018/0250327 A1 (QUIMICA LUAR SRL) 06 September 2018 (2018-09-06) claims, examples 1-4 | 1-10 |
| X | JP 2012-17473 A (THE TRUSTEES OF THE UNIVERSITY OF PENNSYLVANIA) 26 January 2012 (2012-01-26) paragraphs [0015], [0124], examples 1-9 | 1-10 |
| X | JP 2021-11578 A (THE BOEING COMPANY) 04 February 2021 (2021-02-04) claims, paragraphs [0058], [0089], examples | 1-10 |
| X | CN 111518231 A (BEIJING UNIVERSITY OF CHEMICAL TECHNOLOGY) 11 August 2020 (2020-08-11) claims, examples 1-8, test example 1, table 1 | 1, 4, 9-10 |
| X | JP 2007-513959 A (SD PHARMACEUTICALS, INC) 31 May 2007 (2007-05-31) claims, paragraphs [0082]-[0092], antiviral compositions 9, 12, 14, examples 4-5 | 1, 4, 9-10 |
| E, X | JP 2022-41763 A (FANCL CORP) 11 March 2022 (2022-03-11) claims, paragraph [0016], examples, treatment examples 1-2 | 1-10 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **15 April 2022** | **10 May 2022** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**

International application No.
**PCT/JP2022/008180**

**C. DOCUMENTS CONSIDERED TO BE RELEVANT**

| Category* | Citation of document, with indication, where appropriate, of the relevant passages | Relevant to claim No. |
|---|---|---|
| A | JP 2009-526060 A (GOJO INDUSTRIES INC) 16 July 2009 (2009-07-16) claims, paragraphs [0030]-[0031], examples | 1-10 |

Form PCT/ISA/210 (second sheet) (January 2015)

**INTERNATIONAL SEARCH REPORT**
**Information on patent family members**

International application No.
**PCT/JP2022/008180**

| Patent document cited in search report | Publication date (day/month/year) | Patent family member(s) | Publication date (day/month/year) |
|---|---|---|---|
| US 2018/0250327 A1 | 06 September 2018 | WO 2016/185069 A1<br>EP 3295934 A1<br>AR 100459 A<br>MX 2017014162 A<br>BR 112017024399 A | |
| JP 2012-17473 A | 26 January 2012 | US 2006/0024264 A1<br>claims, paragraphs [0107]-[0134], examples 1-9<br>WO 2006/132647 A2<br>EP 1771183 A2<br>CA 2574990 A1<br>KR 10-2007-0058442 A<br>TW 200621268 A | |
| JP 2021-11578 A | 04 February 2021 | US 2018/0362462 A1<br>claims, paragraphs [0089], [0121]-[0173], examples<br>WO 2018/232005 A1<br>EP 3638256 A1<br>CN 110740738 A<br>CA 3065322 A1<br>AU 2018285869 A1<br>BR 112019025924 A<br>JP 2020-523428 A | |
| CN 111518231 A | 11 August 2020 | (Family: none) | |
| JP 2007-513959 A | 31 May 2007 | US 2005/0232895 A1<br>claims, paragraphs [0097]-[0115], antiviral compositions 9, 12, 14, examples 4-5<br>WO 2005/074947 A2<br>EP 1706122 A2<br>CA 2549083 A1<br>KR 10-2006-0118573 A<br>CN 1946410 A<br>AU 2004315262 A1 | |
| JP 2022-41763 A | 11 March 2022 | (Family: none) | |
| JP 2009-526060 A | 16 July 2009 | US 2007/0184013 A1<br>claims, paragraphs [0029]-[0030], examples<br>WO 2007/095008 A2<br>EP 2012838 A2<br>CA 2635270 A1<br>CN 101378787 A<br>AU 2008255166 A1<br>TW 200808330 A<br>KR 10-2008-0108972 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2021011578 A
- JP 2020012214 A


### Non-patent literature cited in the description


- **MANUELA D. et al.** *Antimicrobial Agents and Chemotherapy,* October 2014, vol. 58, 6315-6319
- *POLYMER ENGINEERING AND SCIENCE,* 1974, vol. 14 (2), 147-154